# EUROPÄISCHE PATENTSCHRIFT

(11) **EP 0 508 346 B1**
(45) Veröffentlichungstag und Bekanntmachung des Hinweises auf die Patenterteilung: **17.07.1996**
(21) Anmeldenummer: 92105917.6
(22) Anmeldetag: 06.04.1992
(51) Int. Cl.: C12P 19/18, C12P 19/28, C12P 19/44

(54) **Verfahren zur enzymatischen Synthese von 2-Desoxy-beta-D-galactosiden**
Process for the enzymatic synthesis of 2-desoxy-bêta-D-galactosides
Procédé de synthèse enzymatique de 2-desoxy-bêta-D-galactosides

(30) Priorität: 09.04.1991 DE 4111362
(43) Veröffentlichungstag der Anmeldung: 14.10.1992
(73) Patentinhaber: HOECHST AKTIENGESELLSCHAFT, 65926 Frankfurt am Main (DE)
(72) Erfinder: Thiem, Joachim, Prof. Dr., W-2000 Hamburg (DE); Wiemann, Torsten, W-2000 Hamburg (DE)

(56) Entgegenhaltungen:
- BIOCHEMISTRY, Band 21, 1982, Easton, PA (US); L.J. BERLINER et al., Seiten 6340-6343/
- ANGEWANDTE CHEMIE, INTERNATIONAL ED. Band 30, Nr. 9, September 1991, Weinheim (DE); J. THIEM et al., Seiten 1163-1164/

## Beschreibung

### Verfahren zur enzymatischen Synthese von 2-Desoxy-β-D-galactosiden

Viele komplexe Kohlenhydrate spielen eine zentrale Rolle in biologischen Erkennunsprozessen [G.E. Edelman, Spektrum Wiss. 1984 (6), 62], wie z.B. der Zell-Zell-Erkennung, dem Zellwachstum und der Zelldifferenzierung. Sie konstituieren die Blutgruppendeterminanten [V. Ginsburg, Adv. Enzymol. 36, (1972), 131] und bilden Tumor-assoziierbare Antigene [G.M.W. Cook, E.W. Stoddart, "Surface Carbohydrates of the Eucaryotic Cell", Academic Press, London 1973]. Im Pflanzenbereich können sie als Hormone regulatorische Funktion ausüben [P. Albersheim, A.G. Darvill, Spektrum Wiss. 1985 (11), 86] und bilden die Bindungsstelle für Lektine [T.W. Rademacher, R.B. Parekh, R.A. Dwek, Ann. Rev. Biochem. 57, (1988), 785].

Modifizierte Zucker wie z.B. Desoxy- und Fluorzucker sowie die Epimeren von natürlichen Zuckern stellen wichtige Werkzeuge bei der Erforschung dieser Wechselwirkungen dar. Allgemeine Gesetzmäßigkeiten der Protein-Kohlenhydrat-Wechselwirkung stehen dabei ebenso im Mittelpunkt des Interesses wie spezielle Enzym-Substrat-Wechselwirkungen. So lassen sich beispielsweise durch sukzessive Modifizierung von Enzymsubstraten Aufschlüsse über das aktive Zentrum von Enzymen erhalten. Darüberhinaus finden gerade die Desoxyglycoside durch ihr Auftreten in vielen Antibiotika [T. Reichstein, E. Weiss, Adv. Carbohydr. Chem. 17, (9162), 65] besonderes Interesse.

Spezifische chemische Synthesen von 2-Desoxyglycosiden sind, bedingt durch den fehlenden lenkenden Einfluß eines Substituenten in 2-Position, äußerst schwierig, und ohne lange Synthesesequenzen werden in der Regel *α/β*-Gemische erhalten [J. Thiem, W. Klaffke, Top. Curr. Chem. 154, (1990), 285]. Zudem ist bei solchen Synthesen eine oft aufwendige Schutzgruppenchemie nötig, die eine entsprechende Erhöhung der Stufenzahl und Verringerung der Ausbeute mit sich bringt.

In den letzten Jahren hat die präparative Nutzung von Enzymen zunehmend Anwendung in der Zuckerchemie gefunden. Hohe Stereo- und Regioselektivität bei gleichzeitiger Vermeidung von Schutzgruppenchemie sichern eine, im Vergleich zur chemischen Synthese, gute Ausbeute.

Der Begriff "präparative Nutzung" wird wie folgt definiert: Enzyme mit hoher Substratspezifität sichern zum einen, daß keine störenden Nebenreaktionen ablaufen, zum anderen, daß während der enzymatischen Reaktion keine oder kaum Produkt- bzw. Substrathemmung auftritt, so daß das Reaktionsprodukt in Milligramm- oder Grammengen gewonnen werden kann.

Die Galactosyltransferase EC 2.4.1.22 überträgt Galactosereste regio- und stereospezifisch auf terminale N-Acetylglucosamin-Einheiten, in Anwesenheit von α-Lactalbumin auch auf terminale Glucose, wobei eine β(1-4)-glycosidische Bindung gebildet wird.

Bezüglich einer Variation des Akzeptor-Substrats erlaubt die Spezifität der Transferase einen relativ breiten Spielraum [L.J. Berliner, M.E. Davis, K.E. Ebner, T.A. Beyer, J.E. Bell, Mol. Cell. Biochem. 62, (1984), 37], der in einigen Fällen auch schon präparativ genutzt werden konnte [S.L. Haynie, C.-H. Wong, G.M. Whitesides, J. Org. Chem., 47, (1982), 5416; S. David, C. Auge, Pure Appl. Chem. 59, (1987), 1501; J. Thiem, W. Treder, T. Wiemann, Dechema Biotechnology Conferences, Ed.: D. Behrens, Verlag Chemie, Weinheim, Vol. 2, (1988), 189; J. Thiem; T. Wiemann, Angew. Chem., 102, (1990), 78].

Die Variation des Donor-Substrates ist im Gegensatz zum Akzeptor-Substrat deutlich eingeschränkt. Außer dem natürlichen Substrat wird als einzige weitere Verbindung UDP-4''-Deoxyglucose von der Transferase erkannt (Berliner und Robinson, Biochemistry 1982, 21, S. 6340). Berliner und Robinson weisen das entstandene Reaktionsprodukt nur dünnschichtchromatographisch nach. Über den Ablauf störender Nebenreaktionen oder die Entstehung von Nebenreaktionsprodukten liegen keine Angaben vor, da wahrscheinlich die hierfür eingesetzten Mengen von Substrat und Enzym zu gering sind.

In der europäischen Patentanmeldung 90 11 5862.6 wird ein Verfahren zur enzymatischen Synthese galactosylierter Glycoprotein-Bausteine beschrieben, bei welchem die Galactosyltransferase auch die Synthese von Glycoasparaginen erlaubt.

Es wurde nun gefunden, daß die Galactosyltransferase überraschenderweise auch 2-Desoxy-D-galactosereste übertragen kann und sie in Form der Uridin-5'-diphospho-2-desoxy-D-galactose der Formel II als Donor-Substrat akzeptiert.

Die Erfindung betrifft somit ein Verfahren zur präparativen Synthese der Verbindung der Formel I, 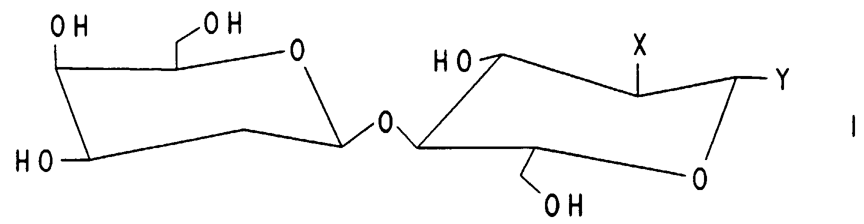 in welcher X = OH oder NH-Acetyl und Y = OH oder 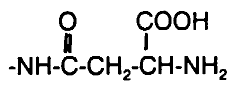 (Asparaginyl) bedeutet, das dadurch gekennzeichnet ist, daß man die Verbindung der Formel II 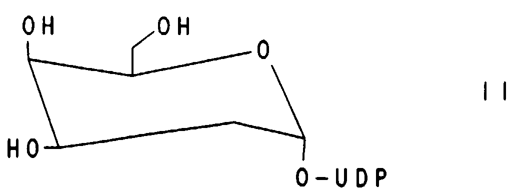 mit der
Verbindung der Formel III, 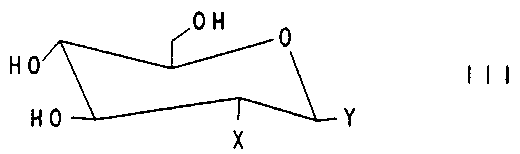 in welcher der Substituent X = OH oder -NH-Ac und Y = OH oder 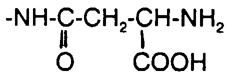 bedeutet,
in Gegenwart des Enzyms Galactosyltransferase umsetzt, wobei die Umsetzung mit Hilfe des Coenzyms Lactalbumin zur Lactose erfolgt, wenn der Substituent X eine Hydroxylgruppe bedeutet.

Im folgenden wird die Erfindung detailliert in ihren bevorzugten Ausführungsformen beschrieben. Ferner wird die Erfindung durch den Inhalt der Ansprüche bestimmt.

Die Galactosyltransferase EC 2.4.1.22, auch als Lactose-Synthetase bezeichnet, akzeptiert Uridin-5'-diphospho-2-desoxy-D-galactose (UDP-2D-Gal) der Formel II als Donor-Substrat, um 2-Desoxygalactose in β(1,4)-glycosidischer Bindung auf terminale N-Acetylglucosamin-, Aspartyl-N-acetylglucosamin- bzw. Glucose-Reste zu übertragen.

Das erfindungsgemäße Verfahren kann in einem sogenannten "Eintopfverfahren" begonnen werden. Unter einem Eintopfverfahren wird verstanden, daß alle beteiligten Substrate und alle beteiligten Enzyme zusammen in einen Reaktionsansatz gegeben werden. Natürlich können die im folgenden genannten Reaktionen auch nacheinander durchgeführt werden. Ein Eintopfverfahren erwies sich jedoch, unter Einhaltung der oben beschriebenen Reaktionspartner für den gesamten Ansatz, als wirtschaftlicher.

UDP-2D-Gal (II) wird vorteilhaft in situ aus 2-Desoxyglucose-6-phosphat hergestellt: 2-Desoxyglucose-6-phosphat wird durch die Phosphoglucomutase EC 2.7.5.1 zu 2-Desoxyglucose-1-phosphat isomerisiert. In Gegenwart von Uridin-5'-diphosphoglucose-Pyrophosphorylase EC 2.7.7.9 wird 2-Desoxyglucose-1-phosphat mit Uridin-5'-triphosphat (UTP) zu Uridin-5'-diphospho-2-desoxyglucose umgesetzt. Durch Hydrolyse des Pyrophosphats mit anorganischer Pyrophosphatase EC 3.6.1.1 wird die durch Uridin-5'-diphosphoglucose-Pyrophosphorylase katalysierte Reaktion in Richtung der gewünschten Produkte verschoben. Uridin-5'-diphospho-2-desoxyglucose wird durch Uridin-5'-diphosphogalactose-4-Epimerase, EC 5.1.3.2 zu UDP-2D-Gal der Formel II epimerisiert, welches in die Transferreaktion auf N-Acetylglucosamin bzw. in Anwesenheit von α-Lactalbumin auf Glucose eingehen kann.

Das bei dieser Reaktion gebildete Uridin-5'-diphosphat (UDP) wird bevorzugt durch Katalyse von Pyruvatkinase EC 2.7.1.40 zu UTP phosphoryliert. Als Phosphatdonor wird Phosphoenolpyruvat (PEP) verwendet. Das UTP kann wieder in die Bildungsreaktion des Nucleotidzuckers Uridin-5'-diphospho-2-desoxyglucose eingehen. Da der Cofaktor UTP laufend regeneriert wird, braucht er lediglich in katalytischen Mengen eingesetzt zu werden.

Die Reaktionsfolge wird vorzugsweise mit Trägern auf modifizierter Kohlenhydratbasis, wie z.B. mit an aktivierte CH-®Sepharose 4B immobilisierten Enzymen durchgeführt [Affinity Chromatography, Principles and Methods, Pharmacia]. Die Immobilisierung erlaubt die Wiedergewinnung der Enzyme in weiteren Reaktionsansätzen.

Als Reaktionsmedium wird Tris-Puffer (100 mM, pH 7.0-8.0, vorzugsweise pH 7,5) verwendet. Der Ansatz wird 5-10 Tage, vorzugsweise 7 Tage bei 28-35°C, vorzugsweise 30°C, inkubiert, anschließend erfolgt die Isolierung des Produktes durch Chromatographie an Gel-lonenaustauschern (Styrol-Divinylbenzol-Copolymerisate, Quervernetzungsgrad 8 %), z.B. ®Dowex 1x8 (Gegenion: Cl⁻) und nachfolgend an porösen, hydrophilen und völlig ladungsfreien Polyacrylamidgelen, z.B. ®Bio-Gel P2. Die Ausbeute beträgt 35 - 40 %.

Das als Ausgangsmaterial für die oben beschriebene Reaktion verwendete 2-Desoxyglucose-6-phosphat wird zuvor aus 2-Desoxyglucose hergestellt. In dieser von der Hexokinase EC 3.6.1.1 katalysierten Reaktion wird als Cofaktor Adenosin-5'-triphosphat (ATP) benötigt. Das gebildete Adenosin-5'-diphosphat (ADP) wird, analog zur oben beschriebenen Regenerierung von UTP, mit Phosphoenolpyruvat (PEP) in Gegenwart von Pyruvatkinase phosphoryliert. Die Hexokinase und die Pyruvatkinase werden vorzugsweise an sphärischen, makroporösen Perlpolymerisaten aus Vinylacetat und Dimethylethylen-Harnstoff, die mit Oxiran-Gruppen modifiziert worden sind, wie z.B. VA-Epoxy (Riedel-de Haen) immobilisiert.

Wenn nicht anders beschrieben, sind alle Ausgangsverbindungen, wie z.B. das Enzym Galactosyltransferase oder die Verbindungen der Formel III, käuflich erhältlich oder können in Anlehnung an die Arbeiten verschiedener Autoren chemisch synthetisiert werden [D.E. Cowley, L. Hough, C.N. Peach, Carbohydr. Res. 19 (1971) 231; M. Spinola, R.W. Jeanloz, J. Biol. Chem. 245 (1970) 4158; H. Kunz, H. Waldmann, Angew. Chem. 97 (1985) 885]. Wird als Akzeptorsubstrat Aspartyl-N-acetylglucosamin eingesetzt, erfolgt die Umsetzung wie in der europäischen Patentanmeldung 90 11 5862.1 beschrieben.

Die Detektion des Reaktionsverlaufs erfolgt dünnschichtchromatographisch. Die Abtrennung des Endproduktes wird nach Entfernung der Nucelotidphosphate durch Ionenaustauschchromatographie, wie z.B. an Dowex 1x8 (Gegenion: Cl⁻), und Gelchromatographie mit beispielsweise Bio-Gel P2® durchgeführt und gelingt in 25 - 35 %iger Ausbeute. Die Ionenaustauscherchromatographie ist damit ein Schritt zur Isolierung des Endprodukts und zur Entfernung von UDP. Da das verwendete Enzym sehr spezifisch die Reaktion katalysiert, fallen keine Nebenreaktionsprodukte an, die einer Entfernung bedürfen. Das Endprodukt läßt sich durch Gelpermeationschromatographie an Bio-Gel P2® eliminieren.

Im folgenden wird die Erfindung mit Hilfe von Beispielen weitergehend erläutert.

### Beispiele

### 1) Synthese von 2-Desoxy-D-glucopyranosyl-1-phosphat (2)

### 1.1) Immobilisierung von Hexokinase (I) und Pyruvatkinase (II) an VA-Epoxy.

| | | | |
|---|---|---|---|
| Immobilisierungspuffer für I: | HEPES | 300 mM | pH 7,5 |
| | Glucose | 25 mM | |
| | ADP | 10 mM | |
| Immobilisierungspuffer für II: | HEPES | 300 mM | pH 7,5 |
| | MgCl₂ | 30 mM | |
| | ADP | 10 mM | |

Der Puffer wird durch Einleiten von Stickstoff vom Sauerstoff befreit. Je 0,5 g VA-Epoxy werden im jeweiligen Puffer suspendiert, anschließend wird, um die Poren des Trägers mit Flüssigkeit zu füllen, vorsichtig Vakuum angelegt. Nach Zugabe von Hexokinase (1000 U) bzw. Pyruvatkinase (1000 U) wird der Ansatz 2 Tage auf dem Schüttelapparat belassen. Nach Filtration und Waschen sind die immobilisierten Enzyme einsatzbereit.
Aktivitätsausbeute: 70 - 80 %.

### 1.2) 2-Desoxy-D-glucopyranosyl-1-phosphat (2)

2-Desoxyglucose (1, 395 mg, 2,4 mmol), Phosphoenolpyruvat (536 mg, 2,6 mmol), Magnesiumchlorid (95 mg, 24 mmol), Kaliumchlorid (300 mg, 100 mmol) und Adenosintriphosphat (12 mg, 20 µmol) werden in 40 ml desoxygeniertem Wasser gelöst und der pH-Wert auf 7,5 eingestellt. Nach Zugabe der an VA-Epoxy immobilisierten Hexokinase und Pyruvatkinase wird der Ansatz auf dem Schüttelapparat belassen. Nach 4 Tagen ist dünnschichtchromatographisch kein Edukt mehr nachweisbar.

| | | |
|---|---|---|
| DC-Kontrolle: | n-Propanol/Ethanol/Wasser | 5:3:2 |
| | R_{f}(1): 0,65 | |
| | R_{f}(2): 0,33 | |

2 wird durch Chromatographie an einer DEAE-Cellulose-Säule (3 x 30 cm), die zuvor mit einer 30 mM Ammoniumhydrogencarbonatlösung äquilibriert worden ist, isoliert. Nach Waschen mit 200 ml Wasser wird das Produkt mit einem Ammoniumhydrogencarbonat-Gradienten (0 - 0,2 M) bei einer Fließgeschwindigkeit von 2 ml/min und einem Totalvolumen von 2 1 eluiert. Die produkthaltigen Fraktionen (2 eluiert bei etwa 30 mM NH₄HCO₃) werden vereinigt und mehrfach lyophilisiert, um das Ammoniumhydrogencarbonat zu entfernen.
Ausbeute: 80 %.

### 2) Darstellung von 2-Acetamido-2-desoxy-4-O-(2-desoxy-β-D-lyxohexopyranosyl)-D-glucopyranose

| | | | |
|---|---|---|---|
| Inkubationspuffer: | Tris | 100 mM | pH 7,5 |
| | MnCl₂ | 5 mM | |
| | MgCl₂ | 10 mM | |
| | KCl | 30 mM | |
| | NaN₃ | 0,02 % | |

In 10 ml desoxygeniertem Puffer werden 2-Desoxyglucose-6-phosphat (2, 60 mg, 190 µmol), N-Acetylglucosamin (6, 71 mg, 321 µmol), UDP (2 mg, 5 µmol), PEP (44 mg, 210 µmol), Glucose-1,6-diphosphat (0,1 mg) und Rinderserumalbumin (BSA, 5 mg) gelöst. Anschließend werden die Enzyme zugegeben:

| | |
|---|---|
| Phosphoglucomutase | 50 U |
| UDP-Glucose-Pyrophosphorylase | 10 U |
| Anorganische Pyrophosphatase | 30 U |
| UDP-Galactose-4-Epimerase | 5 U |
| Galactosyltransferase | 1 U |
| Pyruvatkinase | 100 U |

und der Ansatz 7 d bei 30°C inkubiert.

| | |
|---|---|
| DC-Kontrolle: | n-Propanol/Essigsäure/Wasser 85:12:3 |
| | R_{f} (N-Acetylglucosamin): 0,50 |
| | R_{f} (2-Desoxyglucose): 0,68 |
| | R_{f} (2'-Desoxylactosamin): 0,32 |

Der Umsatz kann entweder durch Phosphatbestimmung nach einer modifizierten Fiske-Subbarow-Methode [T.G. Cooper, "Biochemische Arbeitsmethoden", Walter de Gruyter, Berlin 1981, 53] oder durch enzymatische Bestimmung von Pyruvat [R. Czok, W. Lamprecht, "Methoden der enzymatischen Analyse", Ed.: H.U. Bergmeyer, Verlag Chemie, Weinheim, 1974, Bd. 2, 2. Aufl., 1491] quantifiziert werden.

Die Aufarbeitung erfolgt durch Entfernung der anionischen Bestandteile mit Anionenaustauscher (Dowex 1x8, Cl-Form) und nachfolgender Gelchromatographie an Bio-Gel P2. Nach Gefriertrocknung der produkthaltigen Fraktionen wird das Endprodukt als hygroskopische weiße Substanz mit einem Schmelzpunkt von 136°C erhalten. Eine befriedigende Elementaranalyse läßt sich aufgrund des unbestimmten Wassergehaltes nicht erhalten. Nach azeotroper Trocknung (Aufnehmen in absol. Methanol und mehrfaches Einengen mit absol. Benzol) ist die Substanz analytisch rein.
Ausbeute: 40 % (19 mg).

¹H-NMR (250 MHz, D₂O): δ = 5,26 (d, 1H, H-1, α-Form), 2,18 (m, 1H, H-2'e), 2,09 (s, 3H, NHAc), 1,76 ppm (m, 1H, H-2'a).
Die Signale von H-1 (β-Form) und H-1' werden vom HDO-Signal verdeckt.

¹³C-NMR (63 MHz, H₂O, interner Standart: Dioxan): δ = 173,31 (NH-CO-CH₃), 99,42 (C-1'), 93,93 (C-1, β-Form), 89,59 (C-1, α-Form), 77,87 (C-4, α-Form), 77,44 (C-4, β-Form), 74,65 (C-5'), 73,79 (C-5, β-Form), 71,48 (C-3, β-Form), 69,17 (C-5, α-Form), 68,30 (C-3, α-Form), 66,72 (C-3'), 65,73 (C-4'), 60,43 (C-6'), 59,18 (C-6), 55,29 (C-2, β-Form), 52,80 (C-2, α-Form), 32,54 (C-2'), 21,25 (NHCO-CH₃, β-Form), 20,97 ppm (NHCO-CH₃, α-Form).
C₁₄H₂₅NO₁₀ (367,35)
- FAB-MS:: M+1 = 368
M + Na = 390.

### 3) Synthese von 2'-Desoxylactose

Als Reaktionsmedium wird Tris-Puffer (100 mM, pH 7.5) verwendet. In 4 ml des Puffers werden 50 mg 2-Desoxyglucose-6-phosphat (1, 158 µmol, Dinatriumsalz-Sesquihydrat), 6 mg Uridintriphosphat (10 µmol, Trinatriumsalz-Dihydrat), 36 mg Phosphoenolpyruvat (PEP, 175 µmol, Kaliumsalz) und 43 mg Glucose (5, 235 µmol) gelöst. Nach Kontrolle des pH-Wertes werden 4 mg Manganchlorid (20 µmol), 8 mg Magnesiumchlorid (40 µmol), 11 mg Kaliumchlorid (148 µmol) und eine winzige Menge Natriumazid zugefügt. Anschließend wird durch Einleiten von Helium desoxygeniert. Zum Schluß werden die Proteine zugegeben: 2 mg α-Lactalbumin, 4 mg Rinderserumalbumin (BSA), 100 U Phosphoglucomutase (1), 10 U UDP-Glucose-Pyrophosphorylase (II), 50 U anorganische Pyrophosphatase (III), 10 U UDP-Galactose-4-Epimerase (IV), 5 U Galactosyltransferase (V) und 100 U Pyruvatkinase (VI). Der Ansatz wird bei 37°C, möglichst in einem Schüttelwasserbad, inkubiert.

| | |
|---|---|
| DC-Kontrolle: | DC-Alufolie 60 F254 (Merck). |
| | Laufmittel: n-Propanol/Essigsäure/Wasser |
| | im Verhältnis 85:12:3 |
| | Rf(Glucose, 5): 0.41 |
| | Rf(2'-Desoxylactose, 6): 0.27 |
| | Rf(2-Desoxy-Glc-6-phosphat, 1): 0.05 |
| | Rf(2-Desoxy-Glc): 0.57 (aus Hydrol. von 2) |

Nach ein bis zwei Tagen ist die Produktbildung erkennbar. Es werden noch einmal die Hälfte der oben angegebenen Mengen Uridintriphosphat und der Enzyme zugefügt. Nach weiteren zwei Tagen wird der Ansatz durch Entfernung der Phosphatester auf einer Anionenaustauschersäule (Dowex 1-X8, 200-400 mesh, Gegenion: Cl-, 2.7x20 cm) aufgearbeitet. Das Eluat (2-Desoxyglucose, Glucose und 2'-Desoxylactose) wird lyophilisiert und anschließend durch Gelpermeationschromatographie (Bio-Gel P2, 2x120 cm) aufgetrennt.
Ausbeute: 17 mg (33 %)

## Patentansprüche

1. Verfahren zur präparativen Synthese der Verbindung der Formel I 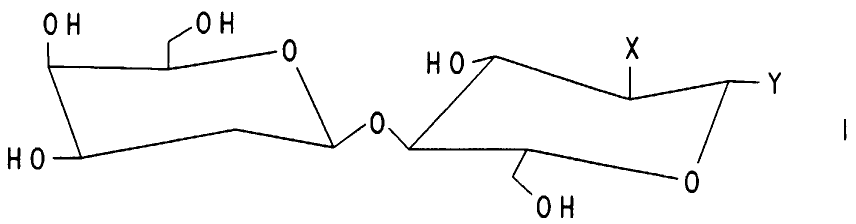 in welcher X = OH oder NH-Ac und Y = OH oder 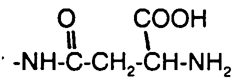 (Asparaginyl) bedeutet, dadurch gekennzeichnet, daß man die Verbindung der Formel II 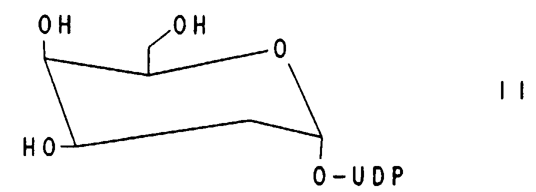 mit der Verbindung der Formel III, 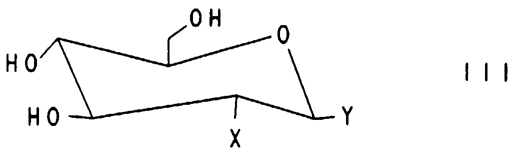 in welcher der Substituent X = OH oder -NH-Ac und Y = OH oder 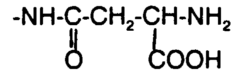 bedeutet, in Gegenwart des Enzyms Galactosyltransferase umsetzt, wobei die Umsetzung mit Hilfe des Coenzyms Lactalbumin zur Lactose erfolgt, wenn der Substituent X eine Hydroxylgruppe bedeutet.

2. Verfahren nach Anspruch 1, dadurch gekennzeichnet, daß die Verbindung der Formel II in situ aus 2-Desoxyglucose-6-phosphat hergestellt und die zu seiner Synthese notwendige Pyruvatkinase EC 2.7.1.40 vorzugsweise in immobilisierter Form verwendet wird.

3. Verfahren nach Anspruch 2, dadurch gekennzeichnet, daß die zur Synthese von 2-Desoxyglucose-6-phosphat verwendete Hexokinase EC 3.6.1.1 in immobilisierter Form eingesetzt wird.

4. Verfahren nach Anspruch 2 oder 3, dadurch gekennzeichnet, daß der Träger für das Immobilisat ein sphärisches, makroporöses Perlpolymerisat aus Vinylacetat und Dimethylethylen-Harnstoff, modifiziert mit Oxiran-Gruppen ist.

5. Verfahren nach Anspruch 1, dadurch gekennzeichnet, daß alle beteiligten Substrate und alle beteiligten Enzyme zusammen in einen Reaktionsansatz gegeben werden.

6. Verfahren nach einem oder mehreren der obengenannten Ansprüche, dadurch gekennzeichnet, daß das Reaktionsmedium einen pH von 7 - 8 hat.

7. Verfahren nach einem oder mehreren der obengenannten Ansprüche, dadurch gekennzeichnet, daß die Reaktionstemperatur 28 - 35°C beträgt.

8. Verfahren nach einem oder mehreren der obengenannten Ansprüche, dadurch gekennzeichnet, daß die Reaktionszeit 5 - 10 Tage beträgt.

## Claims

1. A process for the preparative synthesis of the compound of the formula I 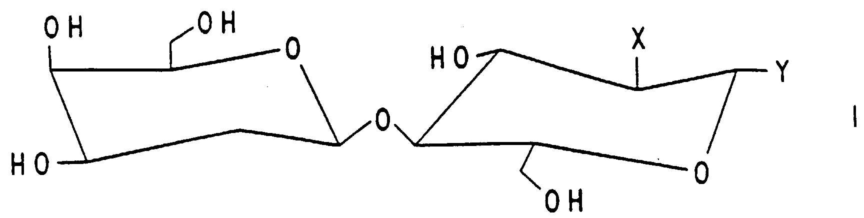 in which X is OH or NH-Ac and Y is OH or 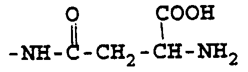 (asparaginyl), which comprises reacting the compound of the formula II 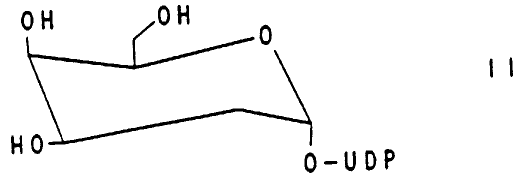 with the compound of the formula III 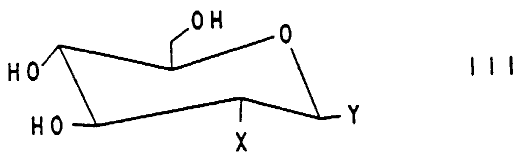 in which the substituent X is OH or -NH-Ac and Y is OH or 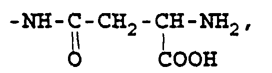 in the presence of the enzyme galactosyltransferase, the reaction being carried out with the aid of the coenzyme lactalbumin to give lactose when the substituent X is a hydroxyl group.

2. The process as claimed in claim 1, wherein the compound of the formula II is prepared in situ from 2-deoxyglucose 6-phosphate, and the pyruvate kinase EC 2.7.1.40 required for its synthesis is preferably used in immobilized form.

3. The process as claimed in claim 2, wherein the hexokinase EC 3.6.1.1 used for the synthesis of 2-deoxyglucose 6-phosphate is employed in immobilized form.

4. The process as claimed in claim 2 or 3, wherein the carrier for the immobilizate is a spherical, macroporous bead polymer of vinyl acetate and dimethylethyleneurea, modified with oxirane groups.

5. The process as claimed in claim 1, wherein all substrates involved and all enzymes involved are put together in one reaction mixture.

6. The process as claimed in one or more of the above-mentioned claims, wherein the reaction medium has a pH of 7 - 8.

7. The process as claimed in one or more of the above-mentioned claims, wherein the reaction temperature is 28 - 35°C.

8. The process as claimed in one or more of the above-mentioned claims, wherein the reaction lasts 5 - 10 days.

## Revendications

1. Procédé de synthèse préparative du composé de formule I 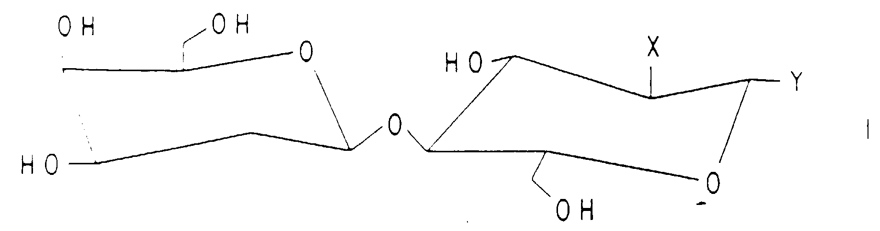 dans laquelle X signifie le groupe hydroxyle ou NH-Ac
et Y = le groupe hydroxyle ou 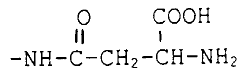 (asparaginyle), caractérisé en ce que l'on fait réagir le composé de formule II 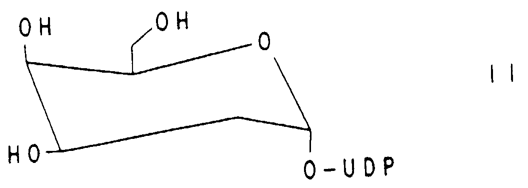 avec le composé de formule III 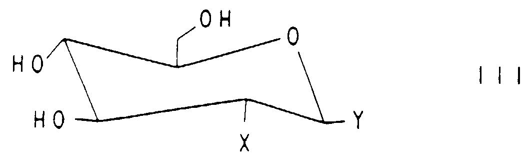 dans lequel le substituant X est OH ou le groupe -NH-Ac et Y = OH ou le groupe 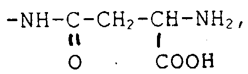 en présence de l'enzyme galactosyl transférase, la réaction à l'aide du coenzyme lactalbumine conduisant au lactose quand le substituant X est un groupe hydroxyle.

2. Procédé selon la revendication 1, caractérisé en ce que, l'on fabrique le composé de formule II in situ à partir du 2-désoxyglucose-6-phosphate et que l'on utilise pour sa synthèse la pyruvate kinase nécessaire EC 2.7.1.40 de préférence sous forme immobilisée.

3. Procédé selon la revendication 2, caractérisé en ce que, pour la synthèse du 2-désoxyglucose-6-phosphate, on utilise l'hexokinase EC 3.6.1.1. sous forme immobilisée.

4. Procédé selon la revendication 2 ou 3, caractérisé en ce que, le support pour l'immobilisat est un polymérisat en forme de perles sphériques, macroporeuses en acétate de vinyle et diméthyléthylène-urée, modifié par des groupes oxiranne.

5. Procédé selon la revendication 1, caractérisé en ce que, tous les substrats et toutes les enzymes concernés sont placés ensemble dans un même mélange réactionnel.

6. Procédé selon une ou plusieurs des revendications susdites, caractérisé en ce que, le milieu réactionnel a un pH de 7-8.

7. Procédé selon une ou plusieurs des revendications susdites, caractérisé en ce que, la température de réaction est de 28-35°C.

8. Procédé selon une ou plusieurs des revendications susdites, caractérisé en ce que, le temps de réaction est de 5 à 10 jours.
